# NEUE EUROPÄISCHE PATENTSCHRIFT

(11) **EP 0 562 263 B2**
(45) Veröffentlichungstag und Bekanntmachung des Hinweises auf die Entscheidung über den Einspruch: **03.02.1999**
(45) Hinweis auf die Patenterteilung: 11.10.1995
(21) Anmeldenummer: 93102444.2
(22) Anmeldetag: 17.02.1993
(51) Int. Cl.: A61K 7/06, A61K 7/08

(54) **Haarwaschmittel**
Hair washing composition
Composition pour le nettoyage des cheveux

(30) Priorität: 06.03.1992 DE 4207046
(43) Veröffentlichungstag der Anmeldung: 29.09.1993
(73) Patentinhaber: GOLDWELL GmbH, D-64297 Darmstadt (DE)
(72) Erfinder: Heinz, Dieter, W-6095 Gustavsburg (DE); Hinz, Sabine, W-6102 Pfungstadt (DE)

(56) Entgegenhaltungen:
- EP-A- 0 186 025
- EP-A- 0 337 354
- EP-A- 0 395 332
- WO-A-92/00058
- WO-A-92/17153
- DE-A- 3 445 919
- DE-A- 3 725 868
- DE-U- 9 110 351
- US-A- 5 045 307
- SEIFEN OLE FETTE WACHSE JOURNAL Bd. 118, Nr. 6, 1. April 1992, AUGSBURG DE Seiten 363 - 368 F. BURMEISTER AND G. J. BROOKS 'vegetable/plant proteins in shampoos'
- Cosmetics and toiletries, 106, 1991, pp. 41-46, Burmeister F. et al
- Cosmetics and toiletries, 105, 1990, p. 100
- CTFA Cosmetic ingredient Handbook, The Cosmetic, Toiletry and Fragance Association Inc, Washington, 1988, p. 337
- Falbe J. und Regitz M. (Hrg.), Römpp Chemie Lexikon, 9. Auflage, Georg Thieme Verlag, Stuttgart, 1989, p. 87
- Firmenschrift von Croda Chemicals Ltd, "Hydrolysed Almond Protein", März 1990
- Firmenschrift von Croda Chemicals Ltd, "Hydrotriticum", Februar 1991
- Ziolkowsky B. (Hrg.) "Emulsionen in Kosmetic und Pharmazie", cosmetik-Jahrbuch 1995, Verlag für chemische industrie H. Ziolkowsky KG, Augsburg, p. 382
- Ziolkowsky B. (Hrg) "Rohstofflexikon", Kosmetikjahrbuch 1990
- Flick E.W. "Cosmetic and toiletry formulations", 1984, Noyes publications, Park Ridge, New Jersey, USA, p. 571

## Beschreibung

Die vorliegende Erfindung betrifft ein Haarwaschmittel, das nicht nur eine gründliche und schonende Reinigung des Haares gewährleistet, sondern gleichzeitig auch noch eine haarpflegende, konditionierende Wirksamkeit aufweist.

Haarwaschmittel mit konditionierenden Eigenschaften sind im Prinzip bekannt. So wurde bereits vorgeschlagen, Shampoos auf Basis üblicher Tenside kationische Polymere, beispielsweise quaternäre Cellulosederivate, zuzusetzen, die sich beim Waschvorgang auf dem Haar ablagern und diesem eine verbesserte Kämmbarkeit und einen weichen Griff sowie ein glänzendes Aussehen verleihen sollen.

Auch der Zusatz von flüchtigen und nicht-flüchtigen Silikonen zu Haarwaschmitteln auf Basis anionischer Tenside ist bereits vorgeschlagen worden. Alle diese Shampoos zeigen zwar eine gewisse Wirkung, vermögen jedoch nicht alle an sie gestellten Anforderungen in ausreichendem Maße zu erfüllen.

Es wurde nun gefunden, und das ist Gegenstand der vorliegenden Erfindung, daß ein Haarwaschmittel auf wäßriger Grundlage sowohl eine gute aber gleichzeitig haarschonende Reinigung und eine exzellente haarpflegende Wirkung im Hinblick auf den Griff, die Kämmbarkeit, die Fülle und den Glanz des Haares ausübt, wenn es ein Gemisch aus mindestens einem anionaktiven Tensid mit mindestens einem Alkylpolyglykosid der allgemeinen Formel RO(R¹O)ₜZₓ, wobei Z einen reduzierenden Saccharidrest mit 5 bis 6 Kohlenstoffatomen, R eine Alkylgruppe mit 8 bis 18 Kohlenstoffatomen, R¹ einen Ethylen- oder Propylenrest, t null bis zehn und x eins bis fünf bedeuten, wobei das Gewichtsverhältnis von anionischen Tensiden zu Alkylpolyglykosid zwischen 10 : 1 und 1 : 5 liegt, in einer Menge zwischen 10 und 30 Gew.-%, bezogen auf die Gesamtzusammensetzung und darüber hinaus ein Gemisch aus haarkonditionierenden Wirkstoffen enthält, das dadurch gekennzeichnet ist, daß es aus 0,1 bis 2,0 Gewichtsprozent mindestens eines kationischen Polymeren und 0,05 bis 2,0 Gew.-% mindestens eines Pflanzenprotein-Hydrolysats besteht, wobei sich die angegebenen Zahlen Jeweils auf die Gesamtzusammensetzung des Haarwaschmittels beziehen.

Das erfindungsgemäße Shampoo ist insbesondere auch dazu geeignet, durch beispielsweise übermäßige Mengen von Oxidationsmittel oder durch anderweitige Einflüsse geschädigtes Haar zu regenerieren, beispielsweise als sogenanntes "Repair-Shampoo".

Als im Rahmen der Erfindung geeignete anionische Tenside sind insbesondere die bekannten C₁₀-C₁₈-Alkylethersulfate zu nennen, insbesondere die C₁₂-C₁₄-Alkylethersulfate bzw. Laurylethersulfat. Ein bevorzugtes Aniontensid besteht aus einem Gemisch aus einem C₁₂-C₁₄-Alkylethersulfat mit beispielsweise 2 bis etwa 10 Ethylenoxid-Gruppen im Molekül, und einem α-Olefinsulfonat, insbesondere einem C₁₂-C₁₆-, vorzugsweise C₁₄-C₁₆-Olefinsulfonat, wobei das bevorzugte Gewichtsverhältnis zwischen Alkylethersulfat und α-Olefinsulfonat im Bereich von etwa 5 : 1 bis etwa 3 : 2 liegt.

Es können selbstverständlich auch andere bekannte anionische Tenside allein oder im Gemisch untereinander eingesetzt werden, beispielsweise Alkalisalze von sulfobernsteinsäurehalbestern, insbesondere das Dinatriumsalz des Monooctylsulfosuccinats und Alkalisalze langkettiger Monoalkylethoxysulfosuccinate.

Weitere geeignete anionaktive Tenside sind Fettalkoholsulfate, beispielsweise Laurylsulfat, Sarkoside, beispielsweise Natriumlaurylsarkosid, Monoglyceridsulfate, Fettsäureamidsulfate, die durch Ethoxylierung und anschließende Sulfatierung von Fettsäuremonoalkanolamiden hergestellt werden, Tauride, Alkylethercarbonsäuren und schließlich Alkalisalze langkettiger Alkylphosphate, die ebenfalls milde, besonders hautverträgliche Detergentien darstellen.
Im Gemisch mit anderen anionischen Tensiden ebenfalls einsetzbar sind Eiweiß-Fettsäure-Kondensationsprodukte an sich bekannter Struktur.
Eine Übersicht über in Shampoos zum Einsatz gelangende anionaktive Tenside findet sich in der Monographie von K. Schrader, 2. Auflage (1989, Hüthig Buchverlag Heidelberg), S. 683 bis 691.

Gemische aus anionaktiven Tensiden und Alkylpolyglykosiden sowie deren Verwendung in Haarwaschmitteln sind an sich bereits bekannt, beispielsweise aus der EP-A 70 074. Die dort beschriebenen Alkylpolyglykoside sind prinzipiell auch im Rahmen der vorliegenden Erfindung geeignet; allerdings wird erfindungsgemäß als besonders bevorzugtes Alkylpolyglykosid ein solches mit etwa 9 bis 11 Kohlenstoffatomen in der Alkylkette und einem Kondensationsgrad von weniger als 1,4, vorzugsweise etwa 1,35, eingesetzt.

Es ist jedoch selbstverständlich, daß auch andere Alkylpolyglykoside, beispielsweise solche mit 12 bis 18 Kohlenstoffatomen in der Alkylkette und höheren Kondensationsgraden, im Rahmen der Erfindung geeignet sind.

Der Anteil an dem Gemisch aus anionaktivem(n) Tensid(en) und Alkylpolyglykosiden(en) in dem erfindungsgemäßen Haarwaschmittel liegt, wie bereits ausgeführt, zwischen etwa 10 und etwa 30 Gewichtsprozent, bezogen auf die Gesamtzusammensetzung des Haarwaschmittels. Vorzugsweise finden etwa 15 bis 25 Gew.-% dieses Gemisches Einsatz.

Das Gewichtsverhältnis von anionischem(n) Tensid(en) zu Alkylpolyglykosid(en) liegt zwischen 10 : 1 und 1 : 5, vorzugsweise 5 : 1 und 1 : 1, insbesondere bei etwa 2 bis 3 : 1.

Gemische aus Sulfosuccinaten und Alkylpolyglykosiden sind aus der EP-A 358 216 bereits bekannt; die dort beschriebenen Gemische eignen sich vorzüglich zum Einsatz in den erfindungsgemäßen Zusammensetzungen.

Die erfindungsgemäßen Haarwaschmittel können selbstverständlich noch weitere Tenside enthalten, insbesondere nichtionische und amphotere.

Gemäß einer weiteren bevorzugten Ausführungsform der Erfindung enthält das Mittel noch ein oberflächenaktives Aminoxid, vorzugsweise in einer Menge von etwa 1 bis etwa 3 Gew.-% der Gesamtzusammensetzung. Ein bevorzugtes Aminoxid ist beispielsweise das Lauryldimethylaminoxid, jedoch können auch andere Aminoxide im Rahmen der Erfindung eingesetzt werden.

Als weitere nichtionische Tensid-Bestandteile können die erfindungsgemäßen Haarwaschmittel beispielsweise langkettige Fettsäuremono- und -dialkanolamide enthalten, beispielsweise Kokosfettsäuremonoethanolamid oder Myristinfettsäurediethanolamid. Der Anteil dieser Alkanolamide liegt vorzugsweise zwischen etwa 0,2 und 2,5, insbesondere 0,5 und 2 Gewichtsprozent der Gesamtzusammensetzung. Solche Gemische sind beispielsweise Gegenstand der EP-A 70 076.

Weitere geeignete nichtionische Tenside sind die verschiedenen Sorbitanester, beispielsweise Polyethylenglykolsorbitanstearinsäureester, Fettsäurepolyglykolester oder Fettsäureglyceridoxethylate, Fettalkoholpolyglykolether und auch Mischkondensate aus Ethylenoxid und Propylenoxid, wie sie unter der Handelsbezeichnung "Pluronics" im Verkehr sind. Diese nichtionischen Tenside sind in der Regel in untergeordneten Mengen, d. h. mit weniger als 2,5 Gew.-% der Gesamtzusammensetzung, enthalten.

Schließlich können, falls erwünscht, in den erfindungsgemäßen Haarwaschmitteln auch amphotere Tenside mitverwendet werden. Solche sind insbesondere die verschiedenen bekannten Betaine, wie Fettsäureamidoalkylbetaine, langkettige Alkylaminopropionate sowie Sulfobetaine. Diese Substanzen und ihre Verwendung in Haarwaschmitteln sind ebenfalls bekannt, beispielsweise aus Schrader, l.c. auf den hierzu verwiesen wird. Ihr Anteil in den erfindungsgemäßen Zusammensetzungen liegt regelmäßig unter 5 Gew.-%, vorzugsweise unter 3 Gew.-% der Gesamtzusammensetzung.

Das haarkonditionierende System nach der Erfindung besteht aus mindestens zwei verschiedenen Bestandteilen.

Der erste essentielle Bestandteil ist ein kationisches Polymeres in einer Menge von 0,1 bis 2,0, vorzugsweise bis 0,5 und besonders bevorzugt von 0,15 bis 0,4 Gewichtsprozent der Gesamtzusammensetzung.

Aus der EP-A 337 354 ist die Verwendung von kationischen Polymeren mit Alkylpolysaccharid-Tensiden an sich bereits bekannt; jedoch läßt sich daraus nicht auf den synergistischen Effekt dieser Bestandteile in der erfindungsgemäßen Zusammensetzung schließen.

Geeignete kationische Polymere sind beispielsweise kationische Cellulosederivate, wie sie unter der Handelsbezeichnung "Polymer JR" vertrieben werden, quaternisierte Mono- und Copolymere von Diallyldimethylammoniumchlorid, quaternäre Copolymerisate des Vinylpyrrolidons, beispielsweise mit Dimethylaminoethylmethacrylat, Polyaminopolyamide, etc. Es wird hierzu, beispielhaft, auf die Aufzählung in der bereits erwähnten EP-A 337 354, S. 3 bis 7, verwiesen.

Der zweite essentielle Bestandteil des haarkonditionierenden Gemisches im Rahmen der vorliegenden Erfindung ist ein Pflanzenprotein-Hydrolysat, das in den Zusammensetzungen in einer Menge von 0,05 bis 2,0, vorzugsweise 0,1 bis 1,0, insbesondere 0,2 bis 0,75 Gewichtsprozent, bezogen auf die Gesamtzusammensetzung, enthalten ist.

Dabei ist das Gewichtsverhältnis des oben definierten kationischen Polymeren und des Pflanzenprotein-Hydrolysats etwa 2 : 1 bis 1 : 2, insbesondere etwa 1 : 1.

Geeignete Pflanzenprotein-Hydrolysate sind insbesondere solche von Glykoproteinen mit einem hohen Anteil an Hydroxyprolin-Gruppen, wie sie insbesondere durch Hydrolyse von Pflanzenzellwandbestandteilen erhalten werden, und die vorzugsweise auch Serin enthalten. Ebenso geeignet sind Pflanzenprotein-Hydrolysate, ausgehend von Sojaprotein, Weizenprotein, Maisprotein, Erdnußprotein, sowie Erbsen, Reis, Korn, Kartoffeln, Bohnen, Cashew , Walnüssen, Erdnüssen und auch Mandelprotein (vgl. EP-A 186 025).

Das Molgewicht dieser Pflanzenprotein-Hydrolysate liegt in der Regel im Bereich zwischen etwa 500 und etwa 100 000, typischerweise zwischen etwa 800 und etwa 50 000, insbesondere zwischen etwa 1 000 und 30 000, besonders bevorzugt zwischen etwa 1 000 und 5 000.
Sie werden durch enzymatische oder Säure-Hydrolyse nach an sich bekannten Verfahren erhalten.

Pflanzenprotein-Hydrolysate werden, im Gegensatz zu Tierproteinhydrolysaten wie Kollagenhydrolysaten etc., in der Kosmetik bisher relativ selten eingesetzt.

Überraschenderweise stellte sich heraus, daß gerade durch die erfindungsgemäße Kombination dieser Pflanzenprotein-Hydrolysate mit kationischen Polymeren eine verbesserte Haarstruktur und mehr Fülle und Volumen des Haares nach einer Behandlung mit dem erfindungsgemäßen Haarwaschmittel erreicht werden als nach Anwendung eines bekannten Produktes mit Eiweißhydrolysaten auf tierischer Basis.

Offenbar bewirkt die Verwendung der pflanzlichen Proteinhydrolysate ein besseres Aufziehen auf das Haar.

Die Eigenschaften des erfindungsgemäßen Haarwaschmittels, insbesondere der Glanz des gewaschenen Haares, können noch verbessert werden durch die Mitverwendung von etwa 0,05 bis etwa 2,5 Gew.-%, bezogen auf die Zusammensetzung des Haarwaschmittels, eines Acyllactylats der allgemeinen Formel 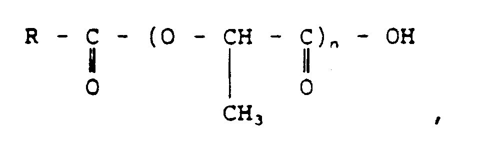 worin R eine gerad- oder verzweigtkettige, gesättigte oder ungesättigte aliphatische Kohlenwasserstoffgruppe mit 6 bis 18 Kohlenstoffatomen darstellt, oder ein wasserlösliches Salz derselben enthält und n eine Zahl zwischen 1 und 5 bedeutet.

Acyllactylate der allgemeinen Struktur 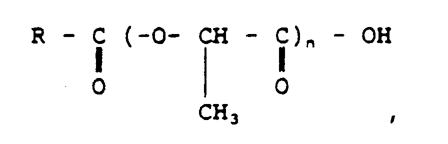 wobei R eine gerad- oder verzweigtkettige Alkyl- oder Alkenylgruppe mit 6 bis 18 Kohlenstoffatomen bedeutet und n für eine Zahl zwischen 1 und 5 steht, und ihre Anwendung in kosmetischen Mitteln, insbesondere als Feuchthaltemittel, sind seit langem bekannt.

Es wird hierzu auf einen Artikel von L.J. Murphy und F.Baiocchi, Drug Cosmetic Industry 122/5 (May 1978), 35, "The Role of Acyl Lactylates in Cosmetics and Toiletries" verwiesen, wonach sie insbesondere als Feuchthaltemittel eingesetzt werden.

In der US-PS 3 275 503 ist die Verwendung dieser Substanzen, insbesondere von Natriumcaproyllactylat, als antimikrobielle Wirkstoffe beschrieben.

Aus der US-PS 3 472 940 ist weiterhin die Verwendung von Acyllactylaten, beispielsweise Natriumstearoyl-2-lactylat, in Deodorant-Stiften bekannt.

Die US-PS 3 728 447 schließlich beschreibt die Verwendung von Fettsäurelactylaten und -glykolaten in Konditionierungs-Shampoos.

Ebenso ist aus der EP-A 278 370 die Verwendung von verzweigtkettigen Acyllactylaten und deren Salzen in kosmetischen Mitteln, insbesondere auch Shampoos und Haarspülungen, bekannt.

Alle diese Druckschriften enthalten jedoch keinerlei Hinweis auf die überraschende Wirkung dieser Verbindungsklasse in Kombinationen mit kationischen Polymeren und Pflanzenprotein-Hydrolysaten bei Anwendung in Haarwaschmitteln zur Verbesserung der Elastizität und des Volumens sowie der Erhöhung des Glanzes des Haares.

Wie bereits ausgeführt, werden als im Rahmen der Erfindung geeignete Acyllactylate sowohl gerad- als auch verzweigtkettige Verbindungen eingesetzt. Besonders bevorzugt sind hierbei Stearoyl- und Isostearoyllactylat; die Herstellung des letzteren und anderer verzweigtkettiger Acyllactylate ist in der bereits erwähnten EP-A 278 370 beschrieben, auf die in diesem Zusammenhang ausdrücklich Bezug genommen wird.

Weitere geeignete Acyllactylate sind das Caproyllactylat und dessen Salze, Decanoyllactylat, Lauroyllactylat, Myristoyllactylat, etc. Die bevorzugte Zahl der Milchsäuregruppen liegt bei n = 1 bis 2, jedoch sind auch höhere Zahlen zwischen 3 und 5 möglich.

Die Zahl der Milchsäuregruppen im Acyllactylat wird durch die eingesetzten Mengenanteile und die Reaktionsbedingungen bestimmt; es wird hierzu auch auf die US-PS 2 733 252 verwiesen.

Eine Gruppe von Acyllactylaten, die zum Einsatz in den erfindungsgemäßen Haarwaschmitteln geeignet sind, werden von der Firma Patco Cosmetic Products, C.J. Patterson Co., unter der Bezeichnung "Pationic" vertrieben.

Wie bereits ausgeführt können neben den Acyllactylaten selbst auch ihre wasserlöslichen Salze verwendet werden. Bevorzugt sind hierbei die Alkalisalze wie das Natrium-, Kalium- und Ammoniumsalz sowie Erdalkalisalze, beispielsweise Calciumacyllactylate; jedoch können auch Alkanolaminsalze zum Einsatz gelangen.

Auch ungesättigte Acyllactylate, wie sie z.B. in der EP-A 443 956 beschrieben sind, können im Rahmen der Erfindung verwendet werden.

Der bevorzugte Anteil an Acyllactylat liegt zwischen etwa 0,05 bis etwa 5 Gew.-%, vorzugsweise 0,1 bis 2,5 Gew.-%, insbesondere bis 1,0 Gew.-%, jeweils berechnet auf die Gesamtzusammensetzung.

Die erfindungsgemäßen Haarwaschmittel können selbstverständlich alle üblichen, in solchen Mitteln zum Einsatz gelangenden Stoffe enthalten. Als solche seien Komplexbildner, Farbstoffe, Konservierungsmittel, pH-Regler, Viskositätsregler, beispielsweise anorganische Salze wie Natriumchlorid oder Natriumsulfat, soweit sie nicht ohnehin in den Tensid-Ausgangsmischungen enthalten sind, Duftstoffe, Perlglanzmittel, Verdickungsmittel, Silikone, etc. genannt. Auch die bekannten tierischen Eiweißhydrolysate können mitverwendet werden. Eine Auflistung solcher Zusatzstoffe findet sich beispielsweise bei Schrader, l.c., S. 677 - 722.

Die folgenden Beispiele dienen der Illustration der Erfindung.

### Beispiel 1:

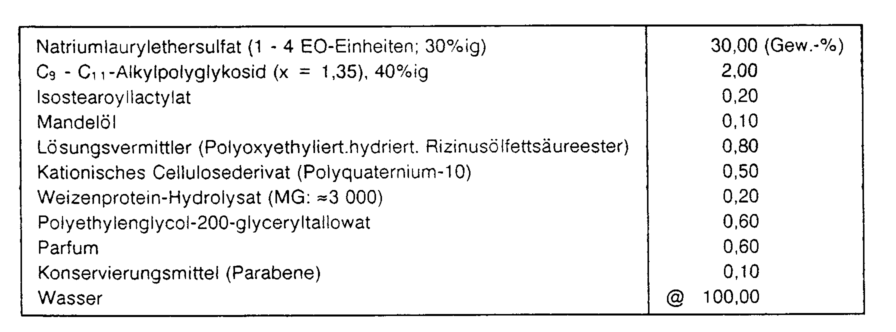

In einem Verbrauchertest an zwei Gruppen mit jeweils 10 Probanden wusch sich eine Gruppe einmal täglich mit einem Shampoo der Zusammensetzung nach Beispiel 1 die Haare, die zweite Gruppe benutzte ein Shampoo gleicher Zusammensetzung, jedoch ohne Weizenprotein-Hydrolysat. Nach acht Tagen wurden die Haare der Probanden verglichen. Es zeigte sich, daß das Haar aller Probanden, die das erfindungsgemäße Shampoo benutzten, deutlich verbesserten Glanz und Spannkraft aufwies sowie lockerer und glatter wirkte als die Haare der Probanden, die das gleiche Shampoo ohne Weizenprotein-Hydrolysat angewandt hatten.

### Beispiel 2:

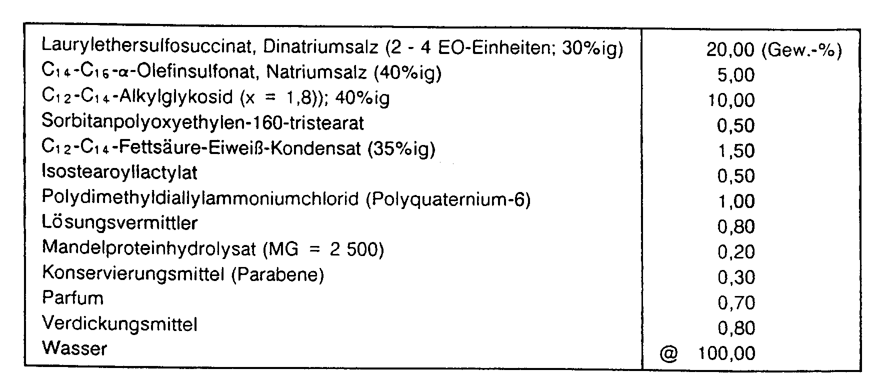

Dieses Shampoo verleiht dem Haar Fülle und Glätte und einen lockeren Griff.

### Beispiel 3:

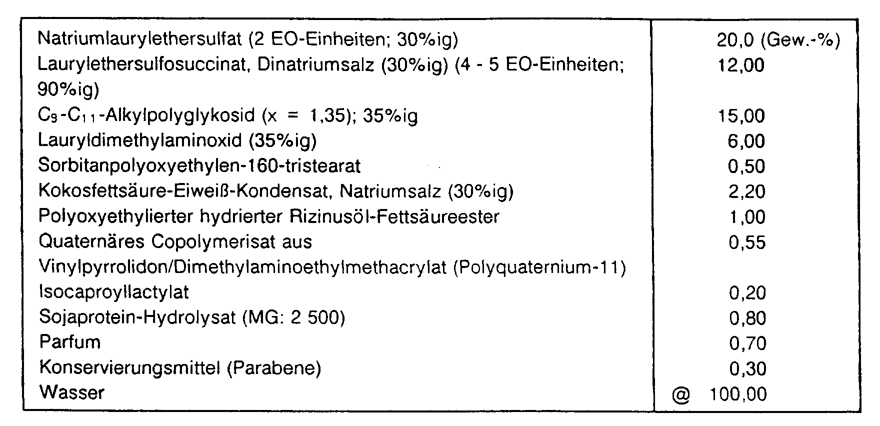

Dieses Shampoo weist die gleichen positiven Eigenschaften wie diejenigen nach den vorhergehenden Beispielen auf.

## Patentansprüche

1. Haarwaschmittel auf wäßriger Grundlage, enthaltend ein Gemisch aus mindestens einem anionaktiven Tensid und mindestens einem Alkylpolyglykosid der allgemeinen Formel 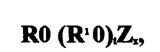 wobei Z einen reduzierenden Sacharidrest mit 5 bis 6 Kohlenstoffatomen, R eine Alkylgruppe mit 8 bis 18 Kohlenstoffatomen, R¹ einen Ethylen- oder Propylenrest, t 0 bis 10 und x 1 bis 5 bedeuten, in einer Menge zwischen 10 und 30 Gew.-%, bezogen auf die Gesamtzusammensetzung, wobei das Gewichtsverhältnis von anionischen Tensiden zu Alkylpolyglykosid zwischen 10:1 und 1:5 liegt, dadurch gekennzeichnet, daß es als haarkonditionierende Wirkstoffe ein Gemisch aus
a) 0,1 bis 2,0 Gew.-%, bezogen auf die Gesamtzusammensetzung, mindestens eines kationischen Polymeren und
b) 0,05 bis 2,0 Gew.-%, bezogen auf die Gesamtzusammensetzung, mindestens eines Pflanzenprotein-Hydrolysats enthält.

2. Haarwaschmittel nach Anspruch 1, dadurch gekennzeichnet, daß es als kationischen Polymeres 0,2 bis 0,5 Gew.-%, bezogen auf die Gesamtzusammensetzung, eines quaternären Cellulosederivats enthält.

3. Haarwaschmittel nach Anspruch 1 oder 2, dadurch gekennzeichnet, daß es als Pflanzenprotein-Hydrolysat 0,2 bis 0,5 Gew.-% bezogen auf die Gesamtzusammensetzung, eines Hydroxyprolin-Gruppen aufweisenden Protein-Hydrolysats enthält.

4. Haarwaschmittel nach einem oder mehreren der Ansprüche 1 bis 3, dadurch gekennzeichnet, daß es 0,05 bis 5 Gew.-%, bezogen auf die Gesamtzusammensetzung, eines Acyllactylats der allgemeinen Formel 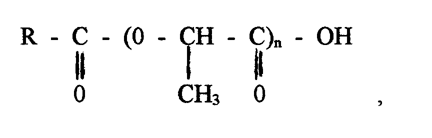 worin R eine gerad- oder verzweigtkettige, gesättigte oder ungesättigte aliphatische Kohlenwasserstoffgruppe mit 6 bis 18 Kohlenstoffatomen darstellt und n eine Zahl zwischen 1 und 5 bedeutet, oder ein wasserlösliches Salz derselben enthält.

5. Haarwaschmittel nach Anspruch 4, dadurch gekennzeichnet, daß es 0,1 bis 1,0 Gew.-%, berechnet auf die Gesamtzusammensetzung, eines Isostearoyl- oder Stearoyllactylats bzw. dessen Alkali- oder Ammoniumsalz enthält.

6. Haarwaschmittel nach einem oder mehreren der Ansprüche 1 bis 5, dadurch gekennzeichnet, daß es das kationische Polymere und das Pflanzenprotein-Hydrolysat im Gewichtsverhältnis von 1:2 bis 2:1 enthält.

7. Haarwaschmittel nach einem oder mehreren der Ansprüche 1 bis 6, dadurch gekennzeichnet, daß es als anionisches Tensid ein Gemisch aus einem C₁₂-C₁₄-Alkylethersulfat und einem α- Olefinsulfonat im Gewichtsverhältnis von 5:1 bis 3:2 enthält.

8. Haarwaschmittel nach einem oder mehreren der Ansprüche 1 bis 6, dadurch gekennzeichnet, daß es als anionisches Tensid ein Alkalisalz eines Sulfobernsteinsäurehalbesters enthält.

9. Haarwaschmittel nach Anspruch 7 oder 8, dadurch gekennzeichnet, daß es als Alkylpolyglykosid ein solches mit einem Kondensationsgrad (x) von weniger als 1,4 enthält.

## Claims

1. Hair shampoo on an aqueous basis, containing a mixture of at least one anion-active surfactant and at least one alkyl polyglucoside of the general formula 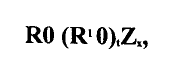 wherein Z stands for a reducing saccharide residue having 5 to 6 carbon atoms, R for an alkyl group of 8 to 18 carbon atoms, R1 for an ethylene or propylene residue, t is zero to ten and x is one to five, in a proportion of 10 to 30 % by weight, calculated to the total composition, whereby the weight proportion of anionic surfactants to alkyl polyglycoside is between 10 : 1 and 1 : 5, characterized in that the hair conditioning substances comprise a mixture of:
a) to 2.0 % by wt., calculated to the total composition, of at least one cationic polymer, and
b) to 2.0 % by wt., calculated to the total composition, of at least one plant protein hydrolyzate.

2. Hair shampoo according to claim 1, characterized in that it comprises, as a cationic polymer, 0.2 to 0.5 % by weight, calculated to the total composition, of a quaternary cellulose derivative.

3. Hair shampoo according to claim 1, characterized in that it comprises, as a plant protein hydrolyzate, 0.2 to 0.5 % by weight, calculated to the total composition, of a protein hydrolyzate containing hydroxyproline groups.

4. Hair shampoo according to one of claims 1 to 3, characterized in that it comprises, calculated to the total composition, 0.05 to 5 % by weight of an acyl lactylate of the general formula: 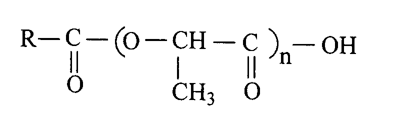 wherein R stands for a straight-chain or branched-chain, saturated or unsaturated aliphatic hydrocarbon group of 6 to 18 carbon atoms, and n is a number between one and five, or water-soluble salts thereof.

5. Hair shampoo according to claim 4, characterized in that it comprises 0.1 to 1.0 % by weight, calculated to the total composition, of an isostearyl or stearyl lactylate or alkali or ammonium salts thereof.

6. Hair shampoo according to one of the preceding claims, characterized in that it comprises the cationic polymer and the plant protein hydrolyzate in a weight proportion of 1 : 2 to 2 : 1.

7. Hair shampoo to one or more of claims 1 to 6, characterized in that it comprises a mixture of a C₁₂-C₁₄-alkyl ether sulfate and an α-olefin sulfonate in a weight proportion of 5 : 1 to 3 : 2.

8. Hair shampoo according to one or more of claims 1 to 6, characterized in that it contains an alkali salt of a sulfosuccinic acid semi-ester.

9. Hair shampoo according to one of claims 7 or 8, characterized in that it comprises a C₉-C₁₁-alkali polyglucoside having a condensation degree (x) of less than 1.4.

## Revendications

1. Composition à base aqueuse pour le lavage des cheveux, contenant un mélange d'au moins un agent tensio-actif anionique et d'au moins un polyglycoside d'alkyle ayant la formule générale 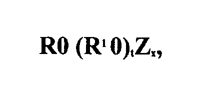 dan laquelle Z représente un reste saccharide réducteur ayant de 5 ou 6 atomes de carbone, R représente un radical alkyle ayant de 8 à 18 atomes de carbone, R¹ représente un radical éthylène ou propylène, t est de 0 à 10 et x de 1 à 5, en une quantité entre 10 et 30 % en poids par rapport à la composition totale, le rapport du poids d'agent tensio-actif anionique au poids du polyglycoside d'alkyle étant entre 10/1 et 1/5, caractérisée en ce qu'elle contient comme agents pour le conditionnement des cheveux un mélange de:
a) de 0,1 à 2,0 % en poids par rapport à la composition totale, d'au moins un polymère cationique, et
b) de 0,05 à 2,0 % en poids par rapport à la composition totale, d'au moins un hydrolysat de protéine végétale.

2. Composition pour le lavage des cheveux selon la revendication 1, caractérisée en qu'elle contient en tant que polymère cationique, de 0,2 à 0,5 % en poids par rapport à la composition totale, d'un dérivé quaternaire de cellulose.

3. Composition pour le lavage des cheveux selon les revendications 1 ou 2, caractérisée en ce qu'elle contient en tant qu'hydrolysat de protéine végétale, de 0,2 à 0,5 % en poids par rapport à la composition totale, d'un hydrolysat de protéine présentant un groupe hydroxyproline.

4. Composition pour le lavage des cheveux selon une ou plusieurs des revendications 1 à 3, caractérisée en ce qu'elle contient de 0,05 à 5 % en poids par rapport à la composition totale, d'un lactylate d'acyle ayant la formule générale 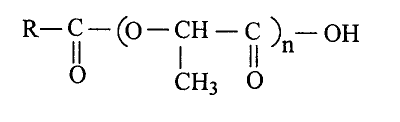 dans laquelle R représente un groupe hydrocarboné aliphatique linéaire ou ramifié, saturé ou non saturé, ayant de 6 à 18 atomes de carbone, et n représente un nombre entre 1 et 5, ou l'un des ses sels hydrosolubles.

5. Composition pour le lavage des cheveux selon la revendication 4, caractérisée en qu'elle contient de 0,1 à 1,0 % en poids par rapport à la composition totale, d'un lactylate d'isostéaryle ou de stéaryle, ou de l'un de leurs sels alcalins ou sels d'ammonium.

6. Composition pour le lavage des cheveux selon une ou plusieurs des revendications 1 à 5, caractérisée en ce qu'elle contient le polymère cationique et l'hydrolysat de protéine végétale dans un rapport en poids de 1/2 à 2/1.

7. Composition pour le lavage des cheveux selon une ou plusieurs des revendications 1 à 6, caractérisée en ce qu'elle contient un mélange d'un éther sulfate d'alkyle en C₁₂-C₁₄ et d'un sulfonate d'α-oléfine dans une proportion pondérale de 5/1 à 3/2, en tant qu'agent tensio-actif anionique.

8. Composition pour le lavage des cheveux selon une ou plusieurs des revendications 1 à 6, caractérisée en ce qu'elle contient en tant qu'agent tensio-actif anionique, un sel alcalin d'un semi-ester de l'acide sulfosuccinique.

9. Composition pour le lavage des cheveux selon l'une des revendications 7 ou 8, caractérisée en ce qu'elle contient en tant que polyglycoside d'alkyle, un polyglycoside d'alkyle en C₉-C₁₁ avec un degré de condensation (x) inférieur à 1,4.
